# EUROPEAN PATENT APPLICATION

(11) **EP 4 768 594 A1**
(43) Date of publication of application: **01.07.2026**
(21) Application number: 25861303.3
(22) Date of filing: 27.08.2025
(51) Int. Cl.: C12P 13/08, C12N 1/20, C12N 1/38, C12R 1/15

(54) **METHOD FOR PRODUCING LYSINE**

(30) Priority: 28.08.2024 KR 20240116275
(71) Applicant: CJ CHEILJEDANG CORPORATION, Seoul 04560 (KR)
(72) Inventor: LEE, Yong-Chan, Seoul 04560 (KR); KWAK, Dong Hun, Seoul 04560 (KR); LEE, Su Jin, Seoul 04560 (KR); KIM, Min Sup, Seoul 04560 (KR); PARK, Jeong Ho, Seoul 04560 (KR); KIM, Jun-Woo, Seoul 04560 (KR); LEE, Kang Hoon, Seoul 04560 (KR); SHIN, Jihyun, Seoul 04560 (KR); PARK, Sang Min, Seoul 04560 (KR)
(74) Representative: Meissner Bolte Partnerschaft mbB
(86) International application number: PCT/KR2025/013041
(87) International publication number: WO 2026/049490

(57) **Abstract**

The present invention relates to a method for producing lysine, comprising a step of culturing a microorganism to prepare a fermentation broth containing lysine; and a step of removing carbon dioxide from the fermentation broth containing lysine using a carbon dioxide stripping tower.

## Description

### TECHNICAL FIELD

The present application relates to a method for a method for producing lysine.

### Cross-Reference to Related Application(s)

This application claims priority to and the benefit of Korean Patent Application No. KR10-2024-0116275, filed on August 28, 2024 in the Korean Intellectual Property Office, the disclosure of which is incorporated herein by reference in its entirety.

### BACKGROUND ART

L-lysine is one of the most frequently-used amino acids in the animal feed. Many literatures disclose methods for producing L-lysine from a fermentation broth containing L-lysine, but these methods often result in low L-lysine content in the final lysine product or require ion exchange processes which increase costs due to the use of chemicals in the process and generate large amounts of wastewater, resulting in a decrease in economic efficiency and a lack of environmental friendliness. Therefore, there is a need to develop a method for producing L-lysine in a more economical and environmentally friendly manner.

(Patent Literature 1) U.S. Patent Application Publication (US 2003-0152633 A1)

### DETAILED DESCRIPTION OF THE INVENTION

### TECHNICAL PROBLEM

One object of the present application is to provide a method for producing lysine.

Another object of the present application is to provide a method for removing carbon dioxide in a lysine fermentation broth.

### TECHNICAL SOLUTION

An aspect provides a method for producing lysine, comprising:
(a) a step of culturing a microorganism to prepare a fermentation broth containing lysine; and
(b) a step of removing carbon dioxide from the fermentation broth containing lysine using a carbon dioxide stripping tower.

The present application will be described in detail as follows. Meanwhile, each of the descriptions and embodiments disclosed herein may also be applied to other descriptions and embodiments. In other words, all combinations of various elements disclosed herein fall within the scope of the present application. Additionally, the scope of the present application is not construed to be limited by the detailed description provided below. Additionally, those of ordinary skill in the art may be able to recognize or confirm, using only conventional experimentation, many equivalents to the particular aspects of the present application described herein. Furthermore, it is also intended that these equivalents be included in the present disclosure.

### Definition of Terms

In the present application, the term "fermentation material" may refer to the result of enzymatic or metabolic decomposition of an organic substance using microorganisms. For example, the fermentation material may include a culture product itself obtained by culturing microorganisms in a culture medium, a culture product where microbial cells (microorganisms, or a part of the microorganisms) have been removed, or a concentrate, dry matter, or lyophilizate of the culture product from which microbial cells have been removed. Additionally, in this case, the term "fermentation broth" may include the entire fermentation material containing lysine produced from the microorganisms, or may be one in which impurities have been removed from the fermentation material.

In the present application, the term "process liquid" may collectively refer to a liquid introduced into each process of producing lysine and/or a liquid after each process has been performed. In one example, the process liquid may refer to a fermentation broth, a concentrate, a filtrate, etc. containing lysine.

In the present application, the term "mother liquor" may refer to a liquid that remains after lysine is separated by performing a separation process and/or a concentration/crystallization process, etc. in a process liquid containing lysine.

The lysine may be lysine granules, lysine liquid, lysine crystals, or lysine powder.

The lysine may be L-lysine.

### Step (a): A step of culturing a microorganism to prepare a fermentation broth containing lysine.

The fermentation broth containing lysine may be obtained by culturing a microorganism that produces lysine. Microorganisms that produce lysine includes both wild-type microorganisms or naturally or artificially genetically modified microorganisms, and it may be a microorganism in which a specific mechanism is weakened or enhanced due to factors such as an insertion of an exogenous gene, or an activity enhancement or inactivation of an endogenous gene, and it may be a microorganism comprising genetic modification of a target protein for a method for producing lysine.

In the present application, the microorganism that produces lysine may be a microorganism naturally having lysine producing ability, or a microorganism in which lysine producing ability is conferred on the microorganism that does not have lysine producing ability, but is not limited thereto. Specifically, in the present application, the microorganism producing lysine, or the microorganism having lysine producing ability, may be a microorganism in which some of the genes in the lysine biosynthesis pathway are enhanced or weakened, or some of the genes in the lysine decomposition pathway are enhanced or weakened. The lysine producing ability of the microorganism of the present application being "enhanced" or "increased" means that the lysine producing ability of the microorganism of the present application is enhanced compared to other microorganisms, parent strains, or unmodified microorganisms other than the microorganism of the present application. As an example, the microorganism of the present application may have lysine producing ability increased by about 1% or more, 2% or more, 5% or more, 10% or more, 100% or more, 200% or more, 500% or more, 1000% or more, 1100% or more, 1200% or more, or 1300% or more, compared to the lysine producing ability of other microorganisms, but is not limited thereto. The term "about" is a range including all of ±0.5, ±0.4, ±0.3, ±0.2, ±0.1, and the like, includes all values in a range equal to or similar to the value following the term "about", but is not limited thereto.

In an embodiment, the microorganism that produces lysine may be *Corynebacterium sp.*

A microorganism of the genus *Corynebacterium* may be one or more selected from the group consisting of *Corynebacterium glutamicum*, *Corynebacterium erythrogenes*, *Corynebacterium crudilactis*, *Corynebacterium deserti, Corynebacterium efficiens*, *Corynebacterium callunae*, *Corynebacterium stationis*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, *Corynebacterium crenatum*, and *Corynebacterium flavescens*, but are not limited thereto.

In an embodiment, the microorganism of the genus *Corynebacterium* may be *Corynebacterium glutamicum.*

The culture of the microorganism that produces lysine may be performed according to suitable medium and culture conditions known in the art. Such a culture process may be easily adjusted and used by those skilled in the art according to the selected strain.

In the present application, the term "culture" means growing the microorganism under appropriately controlled environmental conditions. The culture process of the present application may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

In the present disclosure, the term "medium" means a mixed substance containing nutrients required to culture the microorganism as a main component, and the medium supplies nutrients, growth factors, and the like including water that are indispensable for survival and development. Specifically, as the medium and other culture conditions used for culture of the microorganism of the present application, any one may be used without particular limitation as long as it is a medium used for common culture of microorganisms. However, the microorganism of the present application may be cultured in a common medium containing proper carbon sources, nitrogen sources, phosphorus sources, inorganic compounds, amino acids and/or vitamins, and the like while controlling the concentration of lysine, temperature, pH, and the like under aerobic conditions. In an embodiment, the culture medium for the strain of the genus Corynebacterium may be found can be found in the literature "Manual of Methods for General Bacteriology" by the American Society for Bacteriology (Washington D.C., USA, 1981)".

The carbon sources may include carbohydrates such as glucose, saccharose, lactose, fructose, sucrose, and maltose; sugar alcohols such as mannitol and sorbitol, organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used singly or in a combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in a combination of two or more, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used. In addition to these, amino acids, vitamins and/or suitable precursors, and the like may be contained. These components or precursors may be added to the medium batchwise or continuously, but the manner of addition is not limited thereto.

During the culture of the microorganism, the pH of the medium may be adjusted by adding compounds such as ammonium hydroxide, potassium hydroxide, ammonia, phosphoric acid, and sulfuric acid to the medium in a proper manner. Further, during the culture, foaming may be suppressed by using an antifoaming agent such as fatty acid polyglycol ester. Further, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the method for maintaining the state is not limited thereto. In the culture of the present disclosure, the culture temperature may be maintained at 20 to 45°C, specifically 25 to 40°C, and the microorganism may be cultured for about 10 to 160 hours, but the culture conditions are not limited thereto. Lysine produced through the culture of the present application may be secreted into the medium or may remain in the cells.

The fermentation broth containing lysine may be either a fermentation material obtained by culturing a microorganism producing lysine in a medium, or one in which microbial cells (e.g., microorganisms producing lysine, or a part of the microorganisms, etc.) have been removed from the fermentation material. The removal of the microbial cells from the fermentation broth may be performed by a conventional method such as separation (e.g., centrifugation, etc.) or filtration (e.g., use of microbial cell separation equipment, ceramic membrane filter, etc.).

The fermented broth containing lysine obtained by culturing the microorganism producing lysine may have a lysine concentration of 1 g/l or more, 2 g/l or more, 3 g/l or more, 4 g/l or more, 5 g/l or more, 6 g/l or more, 7 g/l or more, 8 g/l or more, 9 g/l or more, 10 g/l or more, 20 g/l or more, 30 g/l or more, 40 g/l or more, 50 g/l or more, 60 g/l or more, 70 g/l or more, 80 g/l or more, 90 g/l or more, or 100 g/l or more (the upper limit is not particularly limited, and may be, for example, about 100 g/l or less, 200 g/l or less, 500 g/l or less, or 1000 g/l or less.) (unit: weight of lysine/volume of fermentation broth).

The method for producing lysine of the present application may further comprise a step of preparing the microorganism producing lysine, a step of preparing a medium for culture of the microorganism, or a combination of these (in any order), for example, prior to the culture step.

### Step (b): A step of removing carbon dioxide from the fermentation broth containing lysine using a carbon dioxide stripping tower

The step (b) may be a step of removing carbon dioxide from the fermentation broth containing lysine using a carbon dioxide stripping tower.

The carbon dioxide stripping tower may comprise:
(1) an inlet part in which the fermentation broth containing lysine is introduced;
(2) a heat source that generates a heat transfer agent;
(3) a body part that comes into contact with the fermentation broth and the heat transfer agent or in which the thermal energy of the heat transfer agent is transferred to the fermentation broth; and
(4) an outlet part through which the fermentation broth is discharged.

The fermentation broth containing lysine may be introduced into the carbon dioxide stripping tower via the inlet part of the carbon dioxide stripping tower, and discharged via the outlet part.

As the retention time of the fermentation broth containing lysine in the carbon dioxide stripping tower increases, the carbon dioxide in the fermentation broth can be more efficiently removed. In an embodiment, the retention time may mean the difference between the time when the fermentation broth is introduced into the inlet part and the time when the fermentation broth is discharged from the outlet part.

In an embodiment, the fermentation broth containing lysine may be retained in the carbon dioxide stripping tower for 30 minutes or more, 1 hour or more, 2 hours or more, 3 hours or more, 4 hours or more, 5 hours or more, 6 hours or more, 7 hours or more, 8 hours or more, 9 hours or more, 10 hours or more, 11 hours or more, 12 hours or more, 14 hours or more, 16 hours or more, 18 hours or more, 20 hours or more, 22 hours or more, 24 hours or more, 0.5 to 48 hours, 0.5 to 42 hours, 0.5 to 36 hours, 0.5 to 30 hours, 0.5 to 24 hours, 0.5 to 18 hours, 0.5 to 12 hours, 0.5 to 10 hours, 0.5 to 8 hours, 0.5 to 6 hours, 0.5 to 5 hours, 0.5 to 4 hours, 0.5 to 3 hours, 0.5 to 2 hours, 1 to 48 hours, 1 to 42 hours, 1 to 36 hours, 1 to 30 hours, 1 to 24 hours, 1 to 18 hours, 1 to 12 hours, 1 to 10 hours, 1 to 8 hours, 1 to 6 hours, 1 to 5 hours, 1 to 4 hours, 1 to 3 hours, 1 to 2 hours, 2 to 48 hours, 2 to 42 hours, 2 to 36 hours, 2 to 30 hours, 2 to 24 hours, 2 to 18 hours, 2 to 12 hours, 2 to 10 hours, 2 to 8 hours, 2 to 6 hours, 2 to 5 hours, 2 to 4 hours, 2 to 3 hours, 3 to 48 hours, 3 to 42 hours, 3 to 36 hours, 3 to 30 hours, 3 to 24 hours, 3 to 18 hours, 3 to 12 hours, 3 to 10 hours, 3 to 8 hours, 3 to 6 hours, 3 to 5 hours, 3 to 4 hours, 4 to 48 hours, 4 to 42 hours, 4 to 36 hours, 4 to 30 hours, 4 to 24 hours, 4 to 18 hours, 4 to 12 hours, 4 to 10 hours, 4 to 8 hours, 4 to 6 hours, 4 to 5 hours, 5 to 48 hours, 5 to 42 hours, 5 to 36 hours, 5 to 30 hours, 5 to 24 hours, 5 to 18 hours, 5 to 12 hours, 5 to 10 hours, 5 to 8 hours, 5 to 6 hours, 6 to 48 hours, 6 to 42 hours, 6 to 36 hours, 6 to 30 hours, 6 to 24 hours, 6 to 18 hours, 6 to 12 hours, 6 to 10 hours, or 6 to 8 hours, but is not limited thereto.

The fermentation broth containing lysine can efficiently remove carbon dioxide in the fermentation broth as the retention time in the carbon dioxide stripping tower increases, so that the flow rate of the fermentation broth introduced into the inlet part can be appropriately set so as to increase the retention time.

In an embodiment, the fermentation solution containing lysine may be introduced into the inlet part at a flow rate of 1 to 150 ml/min, 1 to 125 ml/min, 1 to 100 ml/min, 1 to 90 ml/min, 1 to 80 ml/min, 1 to 70 ml/min, 1 to 60 ml/min, 1 to 50 ml/min, 5 to 150 ml/min, 5 to 125 ml/min, 5 to 100 ml/min, 5 to 90 ml/min, 5 to 80 ml/min, 5 to 70 ml/min, 5 to 60 ml/min, 5 to 50 ml/min, 10 to 150 ml/min, 10 to 125 ml/min, 10 to 100 ml/min, 10 to 90 ml/min, 10 to 80 ml/min, 10 to 70 ml/min, 10 to 60 ml/min, 10 to 50 ml/min, 15 to 150 ml/min, 15 to 125 ml/min, 15 to 100 ml/min, 15 to 90 ml/min, 15 to 80 ml/min, 15 to 70 ml/min, 15 to 60 ml/min, 15 to 50 ml/min, 20 to 150 ml/min, 20 to 125 ml/min, 20 to 100 ml/min, 20 to 90 ml/min, 20 to 80 ml/min, 20 to 70 ml/min, 20 to 60 ml/min, 20 to 50 ml/min, 30 to 150 ml/min, 30 to 125 ml/min, 30 to 100 ml/min, 30 to 90 ml/min, 30 to 80 ml/min, 30 to 70 ml/min, 30 to 60 ml/min, 30 to 50 ml/min, 40 to 150 ml/min, 40 to 125 ml/min, 40 to 100 ml/min, 40 to 90 ml/min, 40 to 80 ml/min, 40 to 70 ml/min, 40 to 60 ml/min, 40 to 50 ml/min, 50 to 150 ml/min, 50 to 125 ml/min, 50 to 100 ml/min, 50 to 90 ml/min, 50 to 80 ml/min, 50 to 70 ml/min, or 50 to 60 ml/min, but is not limited thereto.

The fermentation broth introduced into the inlet part may come into contact with a heat transfer agent generated from a heat source in the body part. When the fermentation broth comes into contact with the heat transfer agent, the thermal energy of the heat transfer agent can be transferred to the fermentation broth.

The body part may include one or more trays with which the fermentation broth can come into contact. The tray is not limited to a specific form, as long as the fermentation broth containing lysine can efficiently come into contact with the heat transfer agent generated by the heat source, and may include 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 2 or more, 26 or more, 27 or more, 28 or more, 29 or more, 30 or more trays (the upper limit is not particularly limited, and may be, for example, about 100 or less). In a specific embodiment, the body part may include 15 to 25 or 20 trays.

The tray may refer to a structure that promotes contact between the fermentation broth containing lysine and the heat transfer agent to allow efficient transfer of thermal energy.

In an embodiment, the tray may be of a tray type or a packing type.

The tray type may refer to a structure in which one or more trays arranged horizontally in a carbon dioxide stripping tower are stacked in layers. In an embodiment, the heat transfer agent can come into contact with the fermentation broth on the tray while ascending from the bottom to the top, thereby transferring thermal energy to the fermentation broth. The fermentation broth can be collected via an outlet part while flowing down from the top to the bottom.

The tray type may include an internal receiving part in which the fermentation broth and the heat transfer agent can come into contact with each other, a weir formed on one side or an outer circumferential surface of the internal receiving part to receive the fermentation broth, and a downcomer that moves the fermentation broth to the next tray when the fermentation broth is filled above the weir, and specifically, it may be a bubble-cap tray, a sieve tray, a valve tray, a disk and doughnut tray, etc., but is not limited thereto.

The bubble-cap tray has a small cap mounted on the tray, and may have holes formed under the cap. While the fermentation broth flows over the upper end of the tray, the heat transfer agent may have a structure that rises through the opening in the cap and come into contact with the fermentation broth.

The sieve tray may have a structure in which one or more holes (sieves) are formed on the surface of the tray, thereby allowing the heat transfer agent to pass through the holes and come into contact with the fermentation broth.

The valve tray may have a structure in which one or more bands (or valves) are provided on the tray, thereby allowing the heat transfer agent to pass through the bands and come into contact with the fermentation broth. The bands may be opened and closed depending on the flow rate of the heat transfer agent.

The disk and doughnut tray may have a structure in which two units in the shape of a disk and a doughnut are arranged in a crossing manner. The heat transfer agent flows between the doughnuts, and the fermentation broth containing lysine flows over the disk and comes into contact with each other to transfer thermal energy to the fermentation broth.

The packing type tray may refer to a structure in which packing is filled in a carbon dioxide stripping tower, and may be classified into structured packing or random packing. The fermentation broth containing lysine can come into contact with the heat transfer agent while passing between the packings, thereby receiving the transfer of thermal energy. The packings primarily used in the structured packing may be corrugated metal sheets, graphic sheets, plastic nets, ceramic honeycombs, and the like, but are not limited thereto. The packings primarily used in the random packing may be Raschig ring, pall ring, Becktil saddle, interlocking saddle, and the like, but are not limited thereto.

In the detailed description, the terms of "upper", "lower", "front end", "rear end", "upper part", "lower part", "front surface", "rear surface", "upper end", "lower end", and the like may be used herein for illustrative purposes to describe various forms of trays, and the shape and location of the tray are not limited by the terms.

The heat source may generate a heat transfer agent capable of transferring thermal energy to the fermentation broth. The heat transfer agent may be a gas heated by the heat source.

In an embodiment, the heat transfer agent may include water vapor, carbon dioxide, and/or ammonia.

The heat transfer agent may have a temperature suitable for transferring thermal energy to the fermentation broth to strip carbon dioxide in the fermentation broth.

In an embodiment, the temperature of the heat transfer agent may be 75 to 150°C, 75 to 145°C, 75 to 140°C, 75 to 135°C, 75 to 130°C, 75 to 125°C, 75 to 120°C, 75 to 115°C, 75 to 110°C, 75 to 105°C, 75 to 100°C, 75 to 95°C, 75 to 90°C, 80 to 150°C, 80 to 145°C, 80 to 140°C, 80 to 135°C, 80 to 130°C, 80 to 125°C, 80 to 120°C, 80 to 115°C, 80 to 110°C, 80 to 105°C, 80 to 100°C, 80 to 95°C, 80 to 90°C, 85 to 150°C, 85 to 145°C, 85 to 140°C, 85 to 135°C, 85 to 130°C, 85 to 125°C, 85 to 120°C, 85 to 115°C, 85 to 110°C, 85 to 105°C, 85 to 100°C, 85 to 95°C, 85 to 90°C, 90 to 150°C, 90 to 145°C, 90 to 140°C, 90 to 135°C, 90 to 130°C, 90 to 125°C, 90 to 120°C, 90 to 115°C, 90 to 110°C, 90 to 105°C, 90 to 100°C, 90 to 95°C, 95 to 150°C, 95 to 145°C, 95 to 140°C, 95 to 135°C, 95 to 130°C, 95 to 125°C, 95 to 120°C, 95 to 115°C, 95 to 110°C, 95 to 105°C, or 95 to 100°C, but are not limited thereto.

The fermentation broth containing lysine in the carbon dioxide stripping tower makes contact at the body part while the heat transfer agent generated in the heat source is ascending, and therefore, in the case of utilizing this, the fermentation broth with a wider surface area and the high-temperature heat transfer agent can come into contact with each other, so that carbon dioxide can be efficiently removed from the fermentation broth.

In an embodiment, the step of removing the carbon dioxide may be performed by allowing the fermentation broth containing lysine, which is introduced into the upper part of the carbon dioxide stripping tower and has a wider surface area, to come into contact with the heat transfer agent generated by heating the fermentation broth containing lysine at the lower part of the carbon dioxide stripping tower.

The carbon dioxide stripping tower may further include a recovery unit that recovers carbon dioxide stripped while the fermentation broth containing lysine is heated by the heat transfer agent. The recovered carbon dioxide may be reused for a carbon dioxide absorption process in which carbon dioxide is subsequently injected into the fermentation broth containing lysine.

The step (b) may be a step of removing carbon dioxide from a fermentation broth containing lysine by 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more.

In an embodiment, the removal ratio of carbon dioxide may be calculated as "the amount of carbon dioxide removed / the amount of carbon dioxide in the fermentation broth containing lysine." The amount of carbon dioxide may be weight or number of moles, and the removal ratio may be a weight ratio or a molar ratio.

The pH of the fermentation broth (or, L-lysine process liquid) from which carbon dioxide has been removed in the step (b) may be 8.5 to 11, 8.5 to 10.8, 8.5 to 10.75, 8.5 to 10.5, 8.5 to 10.25, 8.5 to 10, 8.5 to 9.75, 8.5 to 9.5, 8.75 to 11, 8.75 to 10.8, 8.75 to 10.75, 8.75 to 10.5, 8.75 to 10.25, 8.75 to 10, 8.75 to 9.75, 8.75 to 9.5, 9 to 11, 9 to 10.8, 9 to 10.75, 9 to 10.5, 9 to 10.25, 9 to 10, 9 to 9.75, 9 to 9.5, 9.25 to 11, 9.25 to 10.8, 9.25 to 10.75, 9.25 to 10.5, 9.25 to 10.25, 9.25 to 10, 9.25 to 9.75, 9.25 to 9.5, 9.5 to 11, 9.5 to 10.8, 9.5 to 10.75, 9.5 to 10.5, 9.5 to 10.25, 9.5 to 10, or 9.5 to 9.75, but is not limited thereto.

By removing carbon dioxide in the fermentation broth containing lysine using the carbon dioxide stripping tower, it is possible to obtain a lysine product containing a high content of lysine while achieving a high carbon dioxide removal ratio in a short period of time.

In the case of removing carbon dioxide in the fermentation broth containing lysine using the carbon dioxide stripping tower, the time required to remove carbon dioxide in the same amount of the fermentation broth can be reduced by 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, compared to the case of removing carbon dioxide in the fermentation broth containing lysine by performing a method of concentrating the fermentation broth and/or a method of directly heating the fermentation broth, so that carbon dioxide can be removed more efficiently.

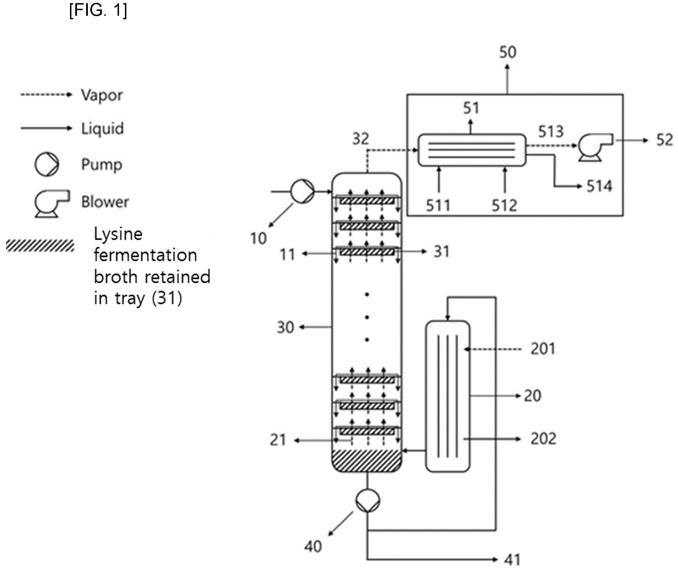

Below, a carbon dioxide stripping tower according to an embodiment will be described in detail with reference to FIG. 1.

Embodiments described in the disclosure and configurations shown in the drawings are merely preferred embodiments of the disclosure, and may be modified in various different ways at the time of filing of this application to replace the embodiments and drawings of the disclosure.

In addition, the same reference numerals or signs shown in the drawings of the disclosure indicate elements or components performing substantially the same function.

Also, the terms used herein are used to describe the embodiments and are not intended to limit and/or restrict the disclosure. The singular forms "a," "an" and "the" are intended to include the plural forms as well, unless the context clearly indicates otherwise. In this disclosure, the terms "including", "having", and the like are used to specify features, numbers, steps, operations, elements, components, or combinations thereof, but do not preclude the presence or addition of one or more of the features, elements, steps, operations, elements, components, or combinations thereof.

In the following detailed description, the terms of "front end", "rear end", "upper part", "lower part", "front surface", "rear surface", "upper end", "lower end", and the like may be defined by the drawings, but the shape and the location of the component is not limited by the term.

In the disclosure, the expression "the same" not only includes complete sameness but also a difference in consideration of a matching error range.

FIG. 1 is a diagram illustrating a carbon dioxide stripping tower according to an embodiment.

Referring to FIG. 1, the carbon dioxide stripping tower may include an inlet part (10) into which a fermentation broth containing lysine is introduced; a heat source (20) that generates a heat transfer agent (21); a body part (30) that comes into contact with the fermentation broth and the heat transfer agent or in which the thermal energy of the heat transfer agent is transferred to the fermentation broth; and an outlet part (40) through which the fermentation broth is discharged.

The input part (10) is a part where the fermentation broth containing lysine is introduced into the body part (30), and may be directly or indirectly connected to the body part. The body part (30) may be connected to 1 or more, 2 or more, 3 or more, 4 or more, or 5 or more inlet parts (10).

The inlet part (10) may be connected to the upper end, middle end, and/or lower end of the body part (30). In an embodiment, the inlet part (10) may be connected to the upper end of the body part (30).

The fermentation broth (11) containing lysine, which is introduced into the body part (30) via the inlet part (10), may come into contact with the body part and receive thermal energy from a heat transfer agent, thereby stripping carbon dioxide in the fermentation broth.

The heat source (20) may generate a heat transfer agent (21) having thermal energy. The heat source (20) may be supplied with thermal energy through steam (201), but is not limited thereto. In an embodiment, the steam (201) supplied to the heat source (20) may be recovered in the form of vapor condensate (202).

The body part (30) may be directly or indirectly connected to the heat source (20). The body part (30) may be connected to one or more, two or more, three or more, four or more, or five or more heat sources (20).

The heat source (20) may be connected at the upper end, middle end, and/or lower end of the body part (30). In an embodiment, the heat source (20) may be connected to the lower end of the body part (30).

The body part (30) may include one or more trays (31) with which the fermentation broth containing lysine may come into contact. As previously described, the tray (31) is not limited to a specific form, as long as the fermentation broth containing lysine may efficiently come into contact with the heat transfer agent (21) generated from the heat source (20).

When the fermentation broth containing lysine receives thermal energy from the heat transfer agent (21), carbon dioxide in the fermentation broth may be stripped.

The outlet part (40) is a part where a fermentation broth (41) containing lysine from which carbon dioxide has been stripped is discharged, and may be directly or indirectly connected to the body part. The body part (30) may be connected to one or more, two or more, three or more, four or more, or five or more recovery parts (40).

The recovery part (40) may be connected to the upper end, middle end, and/or lower end of the body part (30). In an embodiment, the recovery part (40) may be connected to the lower end of the body part (30).

The carbon dioxide stripping tower may further include a recovery part (50) that recovers carbon dioxide stripped while heating the fermentation broth containing lysine by a heat transfer agent.

The recovery part (50) is a part that recovers the stripped carbon dioxide, and may be directly or indirectly connected to the body part. The body part (30) may be connected to one or more, two or more, three or more, four or more, or five or more recovery parts (50).

The recovery part (50) may be connected to the upper end, middle end, and/or lower end of the body part (30). In an embodiment, the recovery part (50) may be connected to the upper end of the body part (30).

The recovery part (50) may be a part into which the gas (32) containing carbon dioxide generated in the body part enters.

The recovery part (50) may include a carbon dioxide separation part (51) and a carbon dioxide recovery device (52).

The internal temperature of the carbon dioxide separation part (51) is reduced by cooling water (511), wherein carbon dioxide (513) can be separated from the gas (32) containing carbon dioxide inside the carbon dioxide separation part. The vapor condensate (514) from which carbon dioxide has been separated may be removed in the carbon dioxide separation part (51).

The separated carbon dioxide (513) is recovered by the carbon dioxide recovery part (52). The recovered carbon dioxide can be reused for the carbon dioxide absorption process in which carbon dioxide is subsequently injected into the fermentation broth containing lysine.

### Step (c): A step of concentrating the lysine process liquid from which carbon dioxide has been removed, obtained in the step (b)

The step (c) may be a step of concentrating the lysine process liquid from which carbon dioxide has been removed, obtained in the step (b).

The step of concentrating the lysine process liquid from which carbon dioxide has been removed, obtained in the step (b), may be a step of concentrating the lysine process liquid until the solid content is 30 to 65 wt.%, 30 to 62.5 wt.%, 30 to 60 wt.%, 30 to 57.5 wt.%, 30 to 55 wt.%, 30 to 52.5 wt.%, 30 to 50 wt.%, 30 to 47.5 wt.%, 30 to 45 wt.%, 30 to 42.5 wt.%, 30 to 40 wt.%, 32.5 to 65 wt.%, 32.5 to 62.5 wt.%, 32.5 to 60 wt.%, 32.5 to 57.5 wt.%, 32.5 to 55 wt.%, 32.5 to 52.5 wt.%, 32.5 to 50 wt.%, 32.5 to 47.5 wt.%, 32.5 to 45 wt.%, 32.5 to 42.5 wt.%, 32.5 to 40 wt.%, 35 to 65 wt.%, 35 to 62.5 wt.%, 35 to 60 wt.%, 35 to 57.5 wt.%, 35 to 55 wt.%, 35 to 52.5 wt.%, 35 to 50 wt.%, 35 to 47.5 wt.%, 35 to 45 wt.%, 35 to 42.5 wt.%, 35 to 40 wt.%, 37.5 to 65 wt.%, 37.5 to 62.5 wt.%, 37.5 to 60 wt.%, 37.5 to 57.5 wt.%, 37.5 to 55 wt.%, 37.5 to 52.5 wt.%, 37.5 to 50 wt.%, 37.5 to 47.5 wt.%, 37.5 to 45 wt.%, 37.5 to 42.5 wt.%, 37.5 to 40 wt.%, 40 to 65 wt.%, 40 to 62.5 wt.%, 40 to 60 wt.%, 40 to 57.5 wt.%, 40 to 55 wt.%, 40 to 52.5 wt.%, 40 to 50 wt.%, 40 to 47.5 wt.%, 40 to 45 wt.%, 40 to 42.5 wt.%, 42.5 to 65 wt.%, 42.5 to 62.5 wt.%, 42.5 to 60 wt.%, 42.5 to 57.5 wt.%, 42.5 to 55 wt.%, 42.5 to 52.5 wt.%, 42.5 to 50 wt.%, 42.5 to 47.5 wt.%, 42.5 to 45 wt.%, 45 to 65 wt.%, 45 to 62.5 wt.%, 45 to 60 wt.%, 45 to 57.5 wt.%, 45 to 55 wt.%, 45 to 52.5 wt.%, 45 to 50 wt.%, 45 to 47.5 wt.%, 47.5 to 65 wt.%, 47.5 to 62.5 wt.%, 47.5 to 60 wt.%, 47.5 to 57.5 wt.%, 47.5 to 55 wt.%, 47.5 to 52.5 wt.%, 47.5 to 50 wt.%, 50 to 65 wt.%, 50 to 62.5 wt.%, 50 to 60 wt.%, 50 to 57.5 wt.%, 50 to 55 wt.%, 50 to 52.5 wt.%, 52.5 to 65 wt.%, 52.5 to 62.5 wt.%, 52.5 to 60 wt.%, 52.5 to 57.5 wt.%, 52.5 to 55 wt.%, 55 to 65 wt.%, 55 to 62.5 wt.%, 55 to 60 wt.%, 55 to 57.5 wt.%, 57.5 to 65 wt.%, 57.5 to 62.5 wt.%, 57.5 to 60 wt.%, 60 to 65 wt.%, or 60 to 62.5 wt.%, but is not limited thereto.

In an embodiment, if the solid content of the lysine process liquid exceeds the above range, the fluidity may decrease, thereby making the process difficult.

The step of concentrating the fermentation broth containing lysine may be a step of concentrating the fermentation broth containing lysine at an atmospheric pressure of 10 to 1000 torr, 10 to 900 torr, 10 to 800 torr, 10 to 700 torr, 10 to 600 torr, 10 to 500 torr, 10 to 400 torr, 10 to 300 torr, 10 to 200 torr, 10 to 100 torr, 20 to 1000 torr, 20 to 900 torr, 20 to 800 torr, 20 to 700 torr, 20 to 600 torr, 20 to 500 torr, 20 to 400 torr, 20 to 300 torr, 20 to 200 torr, 20 to 100 torr, 30 to 1000 torr, 30 to 900 torr, 30 to 800 torr, 30 to 700 torr, 30 to 600 torr, 30 to 500 torr, 30 to 400 torr, 30 to 300 torr, 30 to 200 torr, 30 to 100 torr, 40 to 1000 torr, 40 to 900 torr, 40 to 800 torr, 40 to 700 torr, 40 to 600 torr, 40 to 500 torr, 40 to 400 torr, 40 to 300 torr, 40 to 200 torr, 40 to 100 torr, 50 to 1000 torr, 50 to 900 torr, 50 to 800 torr, 50 to 700 torr, 50 to 600 torr, 50 to 500 torr, 50 to 400 torr, 50 to 300 torr, 50 to 200 torr, or 50 to 100 torr; and/or
at a temperature of 50 to 90°C, 50 to 85°C, 50 to 80°C, 50 to 75°C, 50 to 70°C, 55 to 90°C, 55 to 85°C, 55 to 80°C, 55 to 75°C, 55 to 70°C, 60 to 90°C, 60 to 85°C, 60 to 80°C, 60 to 75°C, 60 to 70°C, 65 to 90°C, 65 to 85°C, 65 to 80°C, 65 to 75°C, or 65 to 70°C, but is not limited thereto.

The step (c) may further comprise a step of injecting carbon dioxide into the lysine concentrate from which the carbon dioxide has been removed.

The carbon dioxide injected in the step (c) may be recycled from the carbon dioxide removed in the step (b).

The method for producing lysine has the advantage that it recycles the carbon dioxide removed in step (b) and does not require a separate carbon source, thereby reducing production costs and being environmentally friendly.

When the step of re-injecting the carbon dioxide is performed, the step (c) may be a step of concentrating the lysine process liquid from which the carbon dioxide has been removed, obtained in the step (b), until the solid content is 30 to 65 wt.%, 30 to 60 wt.%, 30 to 55 wt.%, 30 to 50 wt.%, 30 to 45 wt.%, 35 to 65 wt.%, 35 to 60 wt.%, 35 to 55 wt.%, 35 to 50 wt.%, 35 to 45 wt.%, 40 to 65 wt.%, 40 to 60 wt.%, 40 to 55 wt.%, 40 to 50 wt.%, 40 to 45 wt.%, 45 to 65 wt.%, 45 to 60 wt.%, 45 to 55 wt.%, or 45 to 50 wt.%, but is not limited thereto.

By injecting carbon dioxide into the concentrate, the beneficial effect of improving the hygroscopicity of lysine can be achieved. The term "hygroscopicity" as used herein may mean a tendency to absorb or retain moisture. Since lysine has high hygroscopicity and thus may cause a lumping phenomenon, the hygroscopicity can be improved to prevent the lumping phenomenon.

In an embodiment, the concentrate injected with carbon dioxide may have a molar ratio of anions (in this paragraph, anion may mean bicarbonate ion (HCO₃⁻), carbonate ion (CO₃²⁻), or a combination thereof) to lysine of 0.1 to 52%, 0.1 to 50%, 0.1 to 45%, 0.1 to 41%, 0.1 to 40%, 00.5 to 52%, 0.5 to 50%, 0.5 to 45%, 0.5 to 41%, 0.5 to 40%, 1 to 52%, 1 to 50%, 1 to 45%, 1 to 41%, 1 to 40%, 5 to 52%, 5 to 50%, 5 to 45%, 5 to 41%, 5 to 40%, 10 to 52%, 10 to 50%, 10 to 45%, 10 to 41%, 10 to 40%, 15 to 52%, 15 to 50%, 15 to 45%, 15 to 41%, 15 to 40%, 20 to 52%, 20 to 50%, 20 to 45%, 20 to 41%, 20 to 40%, 25 to 65%, 25 to 52%, 25 to 50%, 25 to 45%, 25 to 41%, 25 to 40%, 30 to 65%, 30 to 52%, 30 to 50%, 30 to 45%, 30 to 41%, 30 to 40%, 35 to 52%, 35 to 50%, 35 to 45%, 35 to 41%, 35 to 40%, 40 to 52%, 40 to 50%, 40 to 45%, or 40 to 41%, but is not limited thereto.

In an embodiment, the concentrate injected with carbon dioxide may have a pH of 8.5 to 10, 8.5 to 9.8, 8.5 to 9.75, 8.5 to 9.5, 8.5 to 9.25, 8.5 to 9, 8.5 to 8.75, 8.75 to 10, 8.75 to 9.8, 8.75 to 9.75, 8.75 to 9.5, 8.75 to 9.25, 8.75 to 9, 9 to 10, 9 to 9.8, 9 to 9.75, 9 to 9.5, 9 to 9.25, 9.25 to 10, 9.25 to 9.8, 9.25 to 9.75, or 9.25 to 9.5, but is not limited thereto.

The step of injecting carbon dioxide may be a step of injecting carbon dioxide using a carbon dioxide absorption tower.

The carbon dioxide absorption tower may comprise:
(1) an inlet part in which the process liquid containing lysine is introduced (in an embodiment, this may be the process liquid from which carbon dioxide has been removed obtained in the step (b), and/or the concentrate in which the process liquid is concentrated);
(2) a carbon dioxide supply device that generates carbon dioxide;
(3) a body part in which the process liquid comes into contact with the carbon dioxide or the carbon dioxide is transferred to the process liquid; and
(4) an outlet part through which the process liquid is discharged.

The (1) inlet part; (3) body part and (4) outlet part are the same as those of the carbon dioxide stripping tower described above.

In an embodiment, the body part may include one or more trays with which the process liquid can come into contact. The trays are not limited to a specific form as long as the process liquid can efficiently come into contact with the carbon dioxide generated in the carbon dioxide supply device, and may include 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, or 30 or more trays (the upper limit is not particularly limited, and may be, for example, about 100 or less). In a specific embodiment, the body part may include 15 to 25 trays or 20 trays.

In an embodiment, the carbon dioxide supply device may supply carbon dioxide that has been removed in the step (b) and then recovered via the recovery part of the carbon dioxide stripping tower.

### Step (d): A step of granulating the concentrate obtained in the step (c)

The step (d) may be a step of granulating the concentrate obtained in the step (c).

In an embodiment, the granulation step may be performed to obtain lysine granule.

The granulation step may be granulated using granulation devices commonly known in the art, such as a dry granulator, wet granulator (low shear granulator (ribbon/paddle mixer, planetary mixer, orbital spiral mixer, sigma blade mixer, etc.), high shear granulator, roller compactor, spray dryer, fluidized bed granulator, and the like.

The lysine granules obtained by performing the granulation step may have a lysine solid content of 75 wt.% or more, 76 wt.% or more, 77 wt.% or more, 78 wt.% or more, 79 wt.% or more, 80 wt.% or more, 80.3 wt.% or more, 80.4 wt.% or more, 81 wt.% or more, 82 wt.% or more, 83 wt.% or more, 84 wt.% or more, 85 wt.% or more, 86 wt.% or more, 87 wt.% or more, 88 wt.% or more, 88.1 wt.% or more, 88.2 wt.% or more, 88.3 wt.% or more; or 75 to 95 wt.%, 75 to 92.5 wt.%, 75 to 90 wt.%, 75 to 89 wt.%, 75 to 88 wt.%, 75 to 87.5 wt.%, 75 to 87 wt.%, 75 to 85 wt.%, 75 to 82.5 wt.%, 75 to 80 wt.%, 77.5 to 95 wt.%, 77.5 to 92.5 wt.%, 77.5 to 90 wt.%, 77.5 to 89 wt.%, 77.5 to 88 wt.%, 77.5 to 87.5 wt.%, 77.5 to 87 wt.%, 77.5 to 85 wt.%, 77.5 to 82.5 wt.%, 77.5 to 80 wt.%, 80 to 95 wt.%, 80 to 92.5 wt.%, 80 to 90 wt.%, 80 to 89 wt.%, 80 to 88 wt.%, 80 to 87.5 wt.%, 80 to 87 wt.%, 80 to 85 wt.%, 80 to 82.5 wt.%, 82.5 to 95 wt.%, 82.5 to 92.5 wt.%, 82.5 to 90 wt.%, 82.5 to 89 wt.%, 82.5 to 88 wt.%, 82.5 to 87.5 wt.%, 82.5 to 87 wt.%, 82.5 to 85 wt.%, 85 to 95 wt.%, 85 to 92.5 wt.%, 85 to 90 wt.%, 85 to 89 wt.%, 85 to 88 wt.%, 85 to 87.5 wt.%, 85 to 87 wt.%, 88 to 95 wt.%, 88 to 92.5 wt.%, 88 to 90 wt.%, 88 to 89 wt.%, 88.1 to 95 wt.%, 88.1 to 92.5 wt.%, 88.1 to 90 wt.%, 88.1 to 89 wt.%, 88.3 to 95 wt.%, 88.3 to 92.5 wt.%, 88.3 to 90 wt.% or 88.3 to 89 wt.%, but is not limited thereto.

The method for a method for producing lysine of the present application may not perform an ion exchange step.

Not performing an ion exchange step may be not performing it in the step (b) of removing carbon dioxide from the fermentation broth containing lysine using a carbon dioxide stripping tower, and also may be not performing it after the step (a) of culturing microorganisms to prepare a fermentation broth containing lysine and/or before the step (c) of concentrating the lysine process liquid from which carbon dioxide has been removed obtained in the step (b).

In the present application, the ion exchange step may mean both a cation exchange step and an anion exchange step. The cation exchange step may mean a step of removing cationic impurities using a cation exchange resin, and the cation exchange resin may mean a commonly known cation exchange resin, such as a strongly acidic cation exchange resin (SAC), a weakly acidic cation exchange resin (WAC), or the like.

The method for producing lysine of the present application does not perform the ion exchange step as described above, and thus eliminates the need for using an ion exchange resin, a pH adjuster, and an eluent, as well as treating the waste liquid, thereby reducing production costs and offering the advantage of being environmentally friendly.

The method for a method for producing lysine of the present application has an advantage that a lysine product containing a high content of lysine can be produced even without performing an ion exchange step.

Another aspect provides a method for removing carbon dioxide in a lysine fermentation broth comprising the steps of:
(a) culturing a microorganism to prepare a fermentation broth containing lysine; and
(b) removing carbon dioxide from the fermentation broth containing lysine using a carbon dioxide stripping tower.

The steps (a), (b), and the like are as described above.

### ADVANTAGEOUS EFFECTS

The present invention relates to a method for producing lysine, comprising a step of culturing a microorganism to prepare a fermentation broth containing lysine; and a step of removing carbon dioxide from the fermentation broth containing lysine using a carbon dioxide stripping tower, wherein carbon dioxide is removed using a carbon dioxide stripping tower, thereby capable of obtaining a lysine product containing a high content of lysine while achieving a high removal ratio of carbon dioxide in a short period of time.

### BRIEF DESCRIPTION OF THE DRAWINGS

FIG. 1 is a diagram illustrating the structure of a carbon dioxide stripping tower according to an embodiment.

### DETAILED DESCRIPTION OF THE EMBODIMENTS

Hereinafter, the present invention will be described in more detail with reference to the examples. However, the following examples are for illustrative purposes only, and are not intended to limit the scope of the present invention.

### Example 1. Preparation of fermentation broth containing L-lysine

*Corynebacterium glutamicum* CJ3P (US 9556463 B2) capable of producing L-lysine was subjected to seed culture in 25 ml of a seed medium at 30 °C and 200 rpm for 20 hours. The microorganism obtained through the seed culture was inoculated at a ratio of 3.0% (v/v) into a 5 L fermentation tank (pH 7.0) containing a medium to which 0.46 ammonium sulfate and 0.03 phosphoric acid were added based on the glucose molar ratio. Culture was performed at 30 °C with sufficient aeration and stirring until the added glucose was completely consumed to thereby obtain the final fermentation broth. The microorganisms obtained through the 5L seed culture were inoculated at a ratio of 20.0% (v/v) into the production medium (30L fermentation tank) containing a medium to which 0.42 ammonium salts (ammonium sulfate, etc.) was added based on the glucose molar ratio. Culture was performed at 30 °C with sufficient aeration and stirring until the added glucose was completely consumed to thereby obtain the final fermentation broth. After the culture was completed, the concentration of L-lysine in the fermentation broth was analyzed using HPLC (Waters, 2478). Microorganisms in the fermentation broth were removed using a membrane having a size of 0.1 µm.

As a result of measuring the concentration of L-lysine in the fermentation broth, the concentration of L-lysine was about 30 g/l.

The compositions of the seed medium (25 ml), seed medium (5 L), and production medium are as follows:
Seed medium (25 ml)
   Based on 1 L of distilled water, 10 g of glucose, 5.0 g of bacto tryptone, 5.0 g of bacto yeast extract, 10.0 g of ammonium sulfate, 2.0 g of urea, 5.0 g of KH₂PO₄, 10.0 g of K₂HPO₄, and 0.5 g of MgSO₄ 7H₂O
Seed medium (5 L)
   Based on 1 L of distilled water, 1 mL of antifoaming agent, 10.0 g of corn steep liquid, 1.0 mg of biotin, 10.0 mg of thiamine, 10.0 mg of pantothenic acid, and 10.0 mg of niacinamide
Production medium
   Based on 1 L of distilled water, 1 mL of antifoaming agent, 10.0 g of corn steep liquid, 1.0 mg of biotin, 10.0 mg of thiamine, 10.0 mg of pantothenic acid, 10.0 mg of niacinamide

### Example 2. Production of lysine granules (carbon dioxide stripping tower process)

### Example 2-1. Production of lysine granules (1)

A carbon dioxide stripping tower was used to remove carbon dioxide in a fermentation broth containing L-lysine. The carbon dioxide stripping tower is a structure including: an upper inlet part into which the fermentation broth containing L-lysine is introduced, one or more trays with which the fermentation broth descends and comes into contact, a lower heat source that generates a heat transfer agent in the upper part, and a lower outlet part from which the fermentation broth is discharged.

In this example, carbon dioxide, present in the fermentation broth containing L-lysine prepared in Example 1, was removed using a carbon dioxide stripping tower (QBR Tech). The carbon dioxide stripping tower includes 20 trays inside, in which the L-lysine process liquid descending from the upper part of the carbon dioxide stripping tower makes gas-liquid contact at each of the 20 trays, while the heat transfer agent heated and generated by a heat source at the lower part of the carbon dioxide stripping tower is ascending. Thus, in the case of utilizing this, the L-lysine process liquid with a wider surface area can come into contact with the hot heat transfer agent, so that carbon dioxide can be efficiently removed from the L-lysine process liquid.

30 kg of the fermentation broth containing L-lysine prepared in Example 1 (solid content: 16 wt.%) was introduced into the carbon dioxide stripping tower so that it is retained for about 4 hours, thereby removing carbon dioxide. Thereby, L-lysine process liquid (about 27.7 kg, pH 10.0, concentration factor 1.08) was obtained in which about 98% of carbon dioxide had been removed.

The removal ratio of carbon dioxide was calculated as "the amount of carbon dioxide removed / the amount of carbon dioxide in the fermentation broth containing lysine", and the removed carbon dioxide was recovered for reuse in a subsequent process through the recovery part in the carbon dioxide stripping tower. The concentration factor reflects the increase in the concentration of lysine due to partial evaporation of water during the process of carbon dioxide removal,and was calculated as "the lysine content in the process liquid discharged after carbon dioxide removal / the lysine content in the fermentation broth introduced".

Table 1 below shows the results of carbon dioxide stripping according to the operation of the carbon dioxide stripping tower in the preparation method of Example 2-1.

**[Table 1]**

| Time | Lower part temperature | Upper part temperature | Bicarbonate concentration | CO₂ removal ratio | Concentration factor |
|---|---|---|---|---|---|
| - | °C | °C | g/L | % | - |
| 1:00 | 103.30 | 97.00 | 0.72 | 98.2 | 1.02 |
| 2:00 | 103.30 | 95.30 | 0.76 | 98.1 | 1.08 |
| 3:00 | 103.00 | 95.30 | 0.59 | 98.5 | 1.04 |
| 4:00 | 103.00 | 95.50 | 0.47 | 98.8 | 1.14 |

27 kg of the L-lysine process liquid prepared by removing the carbon dioxide was introduced into a 20 L rotary evaporator (N-21NS, EYELA) and concentrated until the solid content became 65 wt.% (internal pressure 100 torr, 70°C) to thereby obtain 7.2 kg of L-lysine concentrate (solid content 65 wt.%, pH 10.3, 25°C). The prepared L-lysine concentrate was granulated using a fluidized bed granulator (Daesung Chemical Machinery) to obtain 4.6 kg of L-lysine granules having an L-lysine content of about 88.3%.

### Example 2-2. Production of lysine granules (2) (including carbon dioxide absorption process)

27 kg of the lysine process liquid prepared by removing carbon dioxide in Example 2-1 was introduced into a 20 L rotary evaporator (N-21NS, EYELA) and concentrated until the solid content became 45 wt.% (internal pressure 100 torr, 70°C) to thereby obtain 10.4 kg of L-lysine concentrate (solid content 45 wt.%, pH 10.0, 25°C).

In order to improve the hygroscopicity of the prepared L-lysine concentrate, carbon dioxide was injected in the L-lysine concentrate using a carbon dioxide absorption tower (QBR Tech). The carbon dioxide absorption tower includes 20 trays inside, in which the L-lysine process liquid (L-lysine concentrate) descending from the upper part of the carbon dioxide absorption tower makes gas-liquid contact at each of the 20 trays, while the carbon dioxide generated in the carbon dioxide generator at the lower part of the carbon dioxide absorption tower is ascending. Thus, in the case of utilizing this, the L-lysine process liquid with a wider surface area can come into contact with carbon dioxide, so that carbon dioxide can be efficiently injected into the L-lysine process liquid.

The L-lysine concentrate was injected into the upper part of the carbon dioxide absorption tower at a flow rate of 44.8 ml/min to inject carbon dioxide. The result showed that about 58.4% of the supplied carbon dioxide was absorbed, the pH was 9.0, and the molar ratio of bicarbonate ion/lysine was about 40.3%.

The L-lysine concentrate injected with carbon dioxide was granulated using a fluidized bed granulator (Daesung Chemical Machinery) to obtain 4.7 kg of L-lysine granules having an L-lysine content of about 80.6%. Detailed operating conditions of the fluidized bed granulator are as shown in Example 2-1 and Table 1.

In Comparative Examples 1 and 2 below, a concentration process (Comparative Example 1) or a heating process (Comparative Example 2) was performed to remove carbon dioxide from a fermentation broth containing L-lysine prepared in Example 1. The method for producing L-lysine provided in the present application can obtain a lysine product containing a high content of lysine while achieving a high carbon dioxide removal ratio in a short period of time compared to the production methods of Comparative Examples 1 and 2.

### Comparative Example 1. Production of lysine granules (1) (concentration process)

### Comparative Example 1-1. Production of lysine granules (1)

The fermentation broth containing L-lysine prepared in Example 1 was concentrated to remove carbon dioxide present in the fermentation broth.

30 kg of the fermentation broth containing L-lysine (solid content 16 wt.%) was introduced into a 20 L rotary evaporator (N-21NS, EYELA) and concentrated for 7 hours (internal pressure 100 torr, 70°C) to strip carbon dioxide. Since carbon dioxide was discharged from the fermentation broth, the vacuum inside the concentrator was flexibly adjusted to proceed. As a result of stripping carbonate through the concentration process, carbon dioxide was removed while the fermentation broth containing L-lysine was concentrated. When the fermentation broth was concentrated 1.22 times the input amount (solid content in the fermentation broth: about 19.52 wt.%), about 52% of carbon dioxide was removed, and when the fermentation broth was concentrated 2 times the input amount (solid content in the fermentation broth: 32 wt.%), about 60% of carbon dioxide was removed.

Since the concentration process alone cannot sufficiently remove carbon dioxide, the process liquid was heated with a heating mantle (Daihan Science, DH-WHM12073-EA) to further remove carbon dioxide. The solution was heated three times at 105°C for 2 hours while stirring at 150 rpm using a magnetic stirrer, and a circulator (Julabo, Dyneo DD) was installed on the upper part to re-condense the evaporated water vapor. As a result, 15 kg of L-lysine process liquid from which about 98% of carbon dioxide was removed was obtained.

20 kg of the L-lysine process liquid thus prepared was introduced into a 20 L rotary evaporator (N-21NS, EYELA) and concentrated until the solid content became 65 wt.% (internal pressure 100 torr, 70°C) to thereby obtain 7.38 kg of L-lysine concentrate (solid content 65 wt.%, pH 10.2, 25°C).

The prepared L-lysine concentrate was granulated using a fluidized bed granulator (Daesung Chemical Machinery) to prepare 4.72 kg of L-lysine granules having an L-lysine content of 87.9%.

In this manner, in order to concentrate the fermentation broth containing L-lysine and remove carbon dioxide in the fermentation broth by about 98% or more, a process time of 13 hours (7 hours of concentration + 2 hours of heating * 3 times) or more was required.

### Comparative Example 1-2. Production of lysine granules (2) (including carbonate absorption process)

15 kg of the lysine process liquid prepared in Comparative Example 1-1 was introduced into a 20 L rotary evaporator (N-21NS, EYELA) and concentrated for 7 hours until the solid content became 45 wt.% (internal pressure 100 torr, 70°C) to thereby obtain 10.7 kg of L-lysine concentrate (solid content 45 wt.%, pH 10.2, 25°C).

Carbon dioxide was injected into the prepared L-lysine concentrate using a carbon dioxide absorption tower (QBR Tech). The L-lysine concentrate was injected into the upper part of the carbon dioxide absorption tower of Example 2-2 at a flow rate of 44.8 ml/min to inject carbon dioxide. The result showed that about 58.9% of the supplied carbon dioxide was absorbed, pH was 9.0, and the molar ratio of bicarbonate ion/lysine was about 40.7%.

The L-lysine concentrate injected with carbon dioxide was granulated using a fluidized bed granulator (Daesung Chemical Machinery) to obtain 4.85 kg of L-lysine granules having an L-lysine content of about 80.3%.

### Comparative Example 2. Production of Lysine Granules (2) (Heating Process)

### Comparative Example 2-1. Production of Lysine Granules (1)

The fermentation broth containing L-lysine prepared in Example 1 was heated using a heating mantle to remove carbon dioxide present in the fermentation broth.

30 kg of the fermentation broth containing L-lysine was divided into 5 kg portions in 10 L glass flasks and heated with a heating mantle (Daihan Science, DH-WHM12073-EA). The liquid was heated at 105°C for 4 hours, 6 times while stirring at 150 rpm using a magnetic stirrer, and a circulator (Julabo, Dyneo DD) was installed on the upper part to re-condense the evaporating water vapor. The carbon dioxide stripping ratio according to the heating time is shown in Table 2 below. As a result, 30 kg of L-lysine process solution from which about 98% of carbon dioxide was removed was obtained.

In this manner, when the fermentation broth containing L-lysine was heated using a heating mantle, a heating time of 24 hours (4 hours of heating * 6 times) or more was required to remove about 98% or more of carbon dioxide in the fermentation broth.

30 kg of the prepared L-lysine process liquid was introduced into a 20 L rotary evaporator (N-21NS, EYELA) and concentrated until the solid content became 65 wt.% (internal pressure 100 torr, 70°C) to thereby obtain 7.38 kg of L-lysine concentrate (solid content 65 wt.%, pH 10.3, 25°C).

The prepared L-lysine concentrate was granulated using a fluidized bed granulator (Daesung Chemical Machinery) to obtain 4.8 kg of L-lysine granules having an L-lysine content of about 88.1%.

### Comparative Example 2-2. Production of lysine granules (2) (including carbonate absorption process)

25 kg of the lysine process liquid prepared in Comparative Example 2-1 was introduced into a 20 L rotary evaporator (N-21NS, EYELA) and concentrated until the solid content became 45 wt.% (internal pressure 100 torr, 70°C) to thereby obtain 10.6 kg of L-lysine concentrate (solid content 45 wt.%, pH 10.3, 25°C).

Carbon dioxide was injected into the prepared L-lysine concentrate using the carbon dioxide absorption tower (QBR Tech) of Example 2-2. The L-lysine concentrate was introduced into the upper part of the carbon dioxide absorption tower at a flow rate of 44.8 ml/min to inject carbon dioxide. The result showed that about 59.2% of the supplied carbon dioxide was absorbed, pH was 9.0, and the molar ratio of bicarbonate ion/lysine was about 40.8%.

The L-lysine concentrate injected with carbon dioxide was granulated using a fluidized bed granulator (Daesung Chemical Machinery) to obtain 4.73 kg of L-lysine granules having an L-lysine content of about 80.4%.

From the above description, those skilled in the technical field to which the present invention belongs will understand that the present invention may be implemented in other specific forms without changing the technical spirit or essential features thereof. In this regard, it should be understood that the embodiments described above are illustrative in all respects and not limiting. The scope of the present invention should be construed as including all changes or modified forms derived from the meaning and scope of the claims to be described below rather than the above detailed description, and equivalent concepts thereof.

### [Description of Reference Numerals]

10: inlet part
11: lysine fermentation broth
20: heat source
201: steam
202: vapor condensate
21: heat transfer agent
30: body part
31: tray
32: gas containing carbon dioxide
40: outlet part
41: fermentation broth containing lysine, from which carbon dioxide has been removed
50: recovery part
51: carbon dioxide separation part
511: cooling water in
512: cooling water out
513: carbon dioxide
514: vapor condensate
52: carbon dioxide recovery device

## Claims

1. A method for producing lysine, comprising the steps of:
(a) culturing a microorganism to prepare a fermentation broth containing lysine; and
(b) removing carbon dioxide from the fermentation broth containing lysine using a carbon dioxide stripping tower,
wherein the carbon dioxide stripping tower comprises:
(1) an inlet part in which the fermentation broth containing lysine is introduced;
(2) a heat source that generates a heat transfer agent;
(3) a body part that comes into contact with the fermentation broth and the heat transfer agent or in which thermal energy of the heat transfer agent is transferred to the fermentation broth; and
(4) an outlet part through which the fermentation broth is discharged.

2. The method for producing lysine according to claim 1, wherein the fermentation broth containing lysine in the step (a) is either a fermentation material obtained by culturing lysine-producing microorganism in a medium, or one in which microbial cell has been removed from the fermentation material.

3. The method for producing lysine according to claim 1, wherein the heat transfer agent is a gas.

4. The method for producing lysine according to claim 1, wherein the temperature of the heat transfer agent is 75 to 150 °C.

5. The method for producing lysine according to claim 1, wherein the body part comprises one or more trays with which the fermentation broth can come into contact.

6. The method for producing lysine according to claim 1, wherein the step (b) is a step of removing 85% or more of carbon dioxide from a fermentation broth containing lysine.

7. The method for producing lysine according to claim 1, wherein the pH of fermentation broth containing lysine, from which carbon dioxide has been removed, obtained in the step (b), is 8.5 to 11.

8. The method for producing lysine according to claim 1, wherein the carbon dioxide stripping tower further comprises a recovery part that recovers the stripped carbon dioxide.

9. The method for producing lysine according to claim 1, further comprising a step (c) concentrating the lysine process liquid from which carbon dioxide has been removed, obtained in the step (b).

10. The method for producing lysine according to claim 9, wherein the step (c) is a step of concentrating the lysine process liquid from which carbon dioxide has been removed, obtained in the step (b), until a solid content is 30 to 65 wt.%.

11. The method for producing lysine according to claim 9, further comprising a step (d) granulating a concentrate obtained in the step (c).

12. The method for producing lysine according to claim 11, wherein a lysine content of lysine granule obtained by granulation in the step (d) is 75 to 90 wt.%.

13. The method for producing lysine according to claim 9, wherein the step (c) further comprises injecting carbon dioxide into a lysine concentrate from which the carbon dioxide has been removed.

14. The method for producing lysine according to claim 13, wherein the injecting carbon dioxide is reusing and injecting the carbon dioxide removed in the step (b).

15. The method for producing lysine according to claim 13, wherein the step (c) is a step of concentrating the lysine process liquid from which the carbon dioxide has been removed until the solid content is 30 to 60 wt.%.

16. The method for producing lysine according to claim 13, wherein the concentrate injected with carbon dioxide has a molar ratio of bicarbonate ion (HCO₃⁻) to lysine of 10 to 65%.

17. The method for producing lysine according to claim 13, wherein the concentrate injected with carbon dioxide has a pH of 8.5 to 10.
